# EUROPEAN PATENT SPECIFICATION

(11) **EP 1 378 236 B1**
(45) Date of publication and mention of the grant of the patent: **27.06.2007**
(21) Application number: 02705316.4
(22) Date of filing: 19.03.2002
(51) Int. Cl.: A61K 31/198, A61P 25/00, A61P 25/22, A61P 1/00

(54) **AGENTS AGAINST STRESS-INDUCED DISEASES**
MITTEL GEGEN STRESSINDUZIERTE KRANKHEITEN
AGENTS CONTRE LES MALADIES PROVOQUEES PAR LE STRESS

(30) Priority: 23.03.2001 JP 2001085800; 14.12.2001 JP 2001382190
(43) Date of publication of application: 07.01.2004
(73) Proprietor: Ajinomoto Co., Inc., Tokyo 104-8315 (JP)
(72) Inventor: SMRIGA, M.; c/o Cen. Res. Lab.,AJINOMOTO CO. INC., Kawasaki-shi, Kanagawa 210-0801 (JP); UNEYAMA, H.; c/o Cen. Res. Lab.,AJINOMOTO CO. INC., Kawasaki-shi, Kanagawa 210-0801 (JP); TORII, K.; c/o Cent. Res. Lab., AJINOMOTO CO. INC, Kawasaki-shi, Kanagawa 210-0801 (JP)
(74) Representative: Nicholls, Kathryn Margaret
(86) International application number: PCT/JP2002/002571
(87) International publication number: WO 2002/076445

(56) References cited:
- EP-A- 0 490 379
- EP-A- 0 652 012
- EP-A- 0 728 418
- JP-A- 7 309 750
- DATABASE BIOSIS [Online] BIOSCIENCES INFORMATION SERVICE, PHILADELPHIA, PA, US; June 1999 (1999-06), FIGUEROA J L ET AL: "Lysine and threonine sources for growing pigs under heat stress" XP002303060 Database accession no. PREV200000133926 & CUBAN JOURNAL OF AGRICULTURAL SCIENCE, vol. 33, no. 2, June 1999 (1999-06), pages 183-189, ISSN: 0864-0408
- DATABASE WPI Section Ch, Week 198504 Derwent Publications Ltd., London, GB; Class C03, AN 1985-021708 XP002303062 & JP 59 216551 A (ITO CHU SHIRYO KK) 6 December 1984 (1984-12-06)
- DATABASE BIOSIS [Online] BIOSCIENCES INFORMATION SERVICE, PHILADELPHIA, PA, US; 1984, KATZEN S ET AL: "DIETARY LYSINE EFFECTS ON STRESS-RELATED MORTALITY OF THE MARINE SHRIMP PENAEUS-STYLIROSTRIS" XP002303061 Database accession no. PREV198579002243 & AQUACULTURE, vol. 40, no. 4, 1984, pages 277-282, ISSN: 0044-8486

## Description

### Technical Field

The present invention relates to a novel agent against stress-induced diseases, and a pharmaceutical composition (pharmaceutical product), a food or drink or a feed against stress-induced diseases, particularly for preventing stress-induced diseases. More specifically, the invention relates to an agent (pharmaceutical drug) containing lysine as the active (effective) ingredient, and a pharmaceutical product, a food or drink or a feed, using the agent (pharmaceutical drug). Furthermore, the invention relates to a method for suppressing stress (a method for the therapeutic treatment, amelioration and/or prevention of stress-induced diseases, and/or the like), a use of lysine for anti-stress agents (stress suppressor), and the like.

### Background of the Invention

Lysine is one of essential amino acids in cereals we eat, but is the amino acid of which we most easily fall into deficiency. People mainly eating rice including Japanese people have evaded lysine deficiency, by eating wheat and beans containing lysine, without much ingestion of animal foods. However, people mainly eating corn fall into lysine deficiency, when they don't eat or drink milk or cattle meat. When agricultural production cannot follow the increase of population in future, it is assumed that lysine deficiency may occur mainly in Asia (see Kunio Torii, Clinical Nutrition (Rinsho-Eiyo), 1997, 90(3), 229-232). Additionally, the relation of diseases with nutritious unbalance caused by unbalanced dietary life of aged people and too low dietary intake of juvenile and young men and women, and the like is an issue from the standpoint of nutritional science, although the modern age is called well-fed era. Currently, no detailed survey exists over the nutritious state of people included in those groups and the diseases by which those people are readily affected. It can easily be speculated that those people readily fall into lysine deficiency.

During the course of investigations in terms of nutrition science and health science relating to amino acid, the inventors experimentally found that so-called stress-induced diseases were caused by the lysine deficiency so that resistance against stresses from outer environment was significantly reduced. It was found by using for example the elevated T-maze test of Graeff, et al. (see Graeff F.G. et al., J. Med. Biol. Res., 1993, 26, 67-70) that the anxiety symptoms in rats at lysine deficient state were exacerbated. At a test example in a water-immersion restraint and stress model, it was confirmed that the symptom of gastric ulcer in rats at lysine deficient state was significantly exacerbated.

In such a current complex social environment, on the other hand, it is confirmed that the incidence of stress-induced diseases is high because sensitivity to stresses and the like from outer environment is also increased even at nutritious state with no lysine deficiency.

Stress-induced diseases mean organ disorders of brain (mind) per se or peripheral organs via the effects on the autonomic nervous system or the endocrine system of psychological or physical stressful stimulants (stressors) directly or via the brain emotional system. The relation between stress-induced diseases and actual diseases has not yet been accurately elucidated. In this specification (description), however, stress-induced diseases include neurosis such as anxiety disorders (panic disorders and general anxiety disorders), psychosomatic disorders (somatopathy), dissociated disorders and emotion disorders, and diseases due to psychological stresses, such as abnormal motions of gastric organs (digestive organs) (abnormal gastric (digestive) motion), irritable colon syndrome and gastric (digestive organ) ulcer and additionally includes circulatory disorders such as arrhythmia, angina pectoris, and hypertension, immune disorders such as functional abnormality of lymphocyte, hyperphagia and neurological sitophobia, psilosis, impotency and the like. Furthermore, those to be classified in to the group of psychosomatic diseases are all included therein alike.

As pharmaceutical products for therapeutically treating these stress-induced diseases, a great number of anti-anxiety agents (benzodiazepine derivatives and the like), anti-depression agents (monoamine uptake-inhibiting agents, tricyclic pharmaceutical drugs and the like), and nosotropic pharmaceutical drugs for organic lesions of peripheral organs (for example, antacids, protective agents of gastric mucus, acid secretion suppressors and the like in case of gastric ulcer) have been developed for the purpose of reducing psychological stressors. Although these pharmaceutical products exert certain effects, their effects essentially involve dependency and side effects. Therefore, these pharmaceutical products have not yet produced radical treatment of such diseases. For nutritional improvement, addition of calcium and certain types of vitamins has been attempted. However, no definite therapeutic results have been achieved. Currently, not any agents (pharmaceutical drugs) (pharmaceutical products), foods or drinks, feeds and the like capable of preventing the onset of these stress-induced diseases has been developed yet.

In such circumstances, an agent (pharmaceutical drug) used effectively for stress-induced diseases (agents against (for opposing) stress-induced diseases) and an agent (pharmaceutical drug) capable of preventing these diseases in particular are now needed, which can be incorporated (ingested) safely and can be widely used in pharmaceutical products, foods or drinks, feeds and the like.

In recent years, additionally, clinical practice has paid significant attention to the improvement of quality of life (QOL). Excess psychological stress deteriorates not only the QOL of individual lives after diseases but also the QOL of their daily lives. Such QOL-improving agents for the purpose of improving QOL or agents for preventing such diseases have been demanded strongly in recent years. Numerous companies are under way of the development. However, not any of the companies has achieved the development of a safe agent for the radical therapy thereof.

The effects of pharmaceutical products, foods and/or drinks and feeds on stress-induced diseases, particularly the effects thereof for preventing, ameliorating and therapeutically treating stress-induced diseases can be evaluated in experiments in model rats. The effects on anxiety disorders can be evaluated in models for evaluating anxiety symptoms, using the elevated T-maze test also for use in the evaluation system of anti-anxiety agents. Irritable colon syndrome can be evaluated in wrap stress resistant model (WRS). Further, the evaluation method using water- immersion restraint rat can be applied for assessing the effects on gastric (digestive organ) ulcer. These evaluation methods are widely used as approaches for evaluating the pharmaceutical efficacies of pharmaceutical products for the gastric system (systema digestorium) (see Graeff F.G. et al., J. Med. Biol . Res., 1993, 26, 67-70; Graeff F.G. etal., Pharmacol. Biochem. Behav., 1996, 54, 129-141; Ito C. et al., J. Pharmacol. Exp. Ther., 1997, 280 (1), 67-72; Kishibayashi N. et al., Jpn. J. Pharmacol., 1993, 63, 495-502; Takenaka H. et al., Planta. Med. , 1993, 59, 421-4; Itoh Y. et al., Digestion 1991, 48, 25-33: Tanaka T. et al., Arzneimittelforschung 1993, 43, 558-62).

As one of markers indicating the onset of stress-induced diseases, additionally, serotonin as one of neurotransmitters has been known. Serotonin is now drawing attention as one of neurotransmitters generating emotion in a site responsible for emotion in the brain, namely the amygdala. It is experimentally verified that the elevation of the serotonin concentration in the amygdala in animals such as rat lowers the threshold against anxiety, so that anxiety symptoms are induced, while the reduction of serotonin concentration reduces anxiety-like symptoms at experiments (see Gardner C.R., Pharmacol. Biochem. Behav., 1986, 24, 1479-85; Chung et al., Neuroscience, 2000, 95, 453-63; Kilt et al., Psychopharmacology (Berl), 1981, 74, 290-6). Additionally, a report tells abnormalities in the serotonin system in brain in experimental stress-induced gastric ulcer model (see Hellhammer et al., Psychosom Med. , 1983, 45, 115-22). It is confirmed that pharmaceutical drugs with actions antagonistic to serotonin produce therapeutic effects in stress-induced gastric ulcer model and irritable colon syndrome model (see Mertz HR, Curr Gastroenterol Rep., 1999, 1, 433-40; Camilleri M., Am. J. Med. 1999, 107, 27S-32S; Erin N. et al., Peptides, 1997, 18, 893-8). Based on these findings, it is suggested that the measurement of the serotonin concentration in brain is effective as the approach for evaluating the effect on the prevention of stress-induced diseases.

It has been known that various stresses on animals during feeding and housing cause problems such as the deterioration of culture results in the fields of cattle-raising industries (livestock industries), fishery and culture industries and the like.

### 1. Stress from high-density housing

So as to elevate productivity, generally, chicken, pig, fish and the like are fed and housed at high density. A higher density for feeding and culture causes stress, involving the decrease of the intake of feed, the reduction of immune potency and the like. For example, Nippon Feeding and Culture Standards (for chicken (poultry)), the 1997 version describes on page 60 as follows.

Excessively densified feeding and culture cause stress in chicken, involving the decrease of the productivity, the increase of egg break ratio, the occurrence of cannibalism and the decrease of the survival ratio. Therefore, care should be taken.

### 2. Stress from weaning

So as to elevate productivity, early weaning is done during the feeding and culture of pig. Weaning switches the feed to solid feeds, which causes stresses and involves problems such as the decrease of feed intake.

### 3. Stress during transfer and shipping

Stress from the transfer of the place to be fed and cultured causes the decrease of feed intake. Additionally, the stress from the transfer for shipping causes problems such as the deterioration of meat quality.

These stress problems in the cattle industry and fishery and culture are so serious but not any other approaches except for an approach to improve their feeding environment is found as the method for solving these problems. Currently, therefore, not any approach exists from the standpoint of feed.

As described above, substances with actions against stress-induced diseases are needed, which are applicable as pharmaceutical products and foods or drinks. Even for feeds, further, ingredients with an anti-stress effect on such stresses as described above are also needed, which are to be blended in feeds.

XP002303060 (DATABASE BIOSIS [Online] BIOSCIENCES INFORMATION SERVICE, PHILADELPHIA, PA, US; June 1999 (1999-06), FIGUEROA J L ET AL: "Lysine and threonine sources for growing pigs under heat stress", Database accession no. PREV200000133926) discloses a feed supplemented with lysine. The addition of lysine improved performance of growing pigs under heat stress.

XP002303062 (DATABASE WPI Section Ch, Week 198504 Derwent Publications Ltd., London, GB; Class C03, AN 1985-021708) & JP 59 216551 A (ITO CHU SHIRYO KK) 6 December 1984 (1984-12-06) relates to preventing gizzard ulcer in chicken. The ulcer is apparently caused by "GE-substance" (a heat denatured complex of free histidine and protein).

XP002303061 (DATABASE BIOSIS [Online] BIOSCIENCES INFORMATION SERVICE, PHILADELPHIA, PA, US; 1984, KATZEN S ET AL: "DIETARY LYSINE EFFECTS ON STRESS-RELATED MORTALITY OF THE MARINE SHRIMP PENAEUS-STYLIROSTRIS", Database accession no. PREV198579002243) discloses that lysine was used in the treatment of a disease of organic (probably viral) origin.

EP-A-0 728 418 describes the use of lysine along with other amino acids to improve the physical strength of dairy cows around the time of calving.

EP-A-0 652 012 describes a composition including sugars and amino acids as a base material to enhance the transportation of other materials past the blood-brain barrier. The base material may contain lysine as one of the amino acids, but need not do so.

### Problems that the Invention is to Solve

The problems to be solved by the invention are to develop a pharmaceutical drug effective for a group of diseases generically called stress-induced diseases as described above (agent against stress-induced diseases), particularly a pharmaceutical drug capable of preventing the group of such diseases and to provide a pharmaceutical composition, a food or drink or a feed using the pharmaceutical drug (agent).

### Disclosure of the Invention

The present inventors made diligent investigations in such circumstances. Consequently, the inventors have found that stress-induced diseases easily occur due to lysine deficiency on the background of food conditions and additionally that stress-induced diseases frequently occur even in normal subjects without lysine deficiency in these modern social environment as well. As the results of further investigations, the inventors have found that sufficient lysine supplementation in an immediate fashion in cases at lysine-deficient nutritious states and preliminary supplementation of sufficient lysine in cases without lysine deficiency are effective for stress-induced diseases. Particularly, the inventors have found that lysine supplementation can prevent neuroses such as anxiety disorders (panic disorders and general anxiety disorders), psychosomatic disorders, dissociated disorders and emotion disorders, and abnormalities in gastric motions (abnormal gastric (digestive) motion), irritable colon syndrome (irritable bowel syndrome) and gastric (digestive organ) ulcer and that such lysine supplementation can therapeutically treat and ameliorate the diseases readily, even at the onset. In other words, the inventors have found that preliminary intake of sufficient lysine can produce the preventive effect and that sufficient lysine intake can produce the treatment and amelioration effects, even at the onset.

The inventors have also found that lysine can produce an effect on (competitive with) the stresses intrinsic to animals, when the level of lysine in feeds is set at higher values.

Based on these various findings, the invention has been achieved.

In accordance with the invention, lysine is used as an active ingredient in the manufacture of a therapeutic composition against stress-induced diseases, as specified below, for therapeutic treatment, amelioration or prevention thereof. The therapeutic composition is selected from pharmaceutical compositions, foods, drinks and feeds.

In other words, in one aspect, the invention relates to a pharmaceutical composition, a food or drink or a feed for opposing stress-induced diseases, characteristically containing lysine.

The lysine may be used in the free form but may also be used in the form of salt(s), or the form of their mixture(s) (including a mixture with plural such salts and a mixture of the free form with one or more types of such salts) (in this specification (description), the free form and its salt form(s) are collectively called "lysine"). Further, the L form is adopted because it can be metabolized in biological organisms.

The term "for opposing stress-induced diseases" meaningfully includes those widely used to oppose stress-induced diseases and includes the prevention, amelioration, progress blocking, and therapeutic treatment of stress-induced diseases. Lysine can preferably be used particularly for the prevention of stress-induced diseases.

Any component containing at least lysine and exerting the pharmacological effects as described above may be satisfactory as the component to be contained in such agents or foods or drinks or feeds. Unless other components inhibit the effects of the invention, the components may be contained in such agents or foods or drinks or feeds.

The other components are preferably amino acid(s) (one type or plural types). Particularly preferably, specific amino acid(s) (which may be in salt form(s)) such as arginine (L-arginine), glutamic acid (L-glutamic acid), and aspartic acid (L-aspartic acid) can be contained or used therein. In this case, one type or plural types may be used. Such amino acids are particularly useful for (to oppose) ulcer and anxiety. For use, lysine and arginine may be used in the form of salts with glutamic acid and aspartic acid.

As described above, lysine may be used in the free form, but may also be used in salt form(s). In this case, any acid may be used for composing such salts, with no specific limitation, but includes for example inorganic acids such as hydrochloric acid, sulfuric acid and carbonic acid, and organic acids such as amino acids including glutamic acid and aspartic acid, acetic acid and acetylsalicylic acid.

Particularly preferably, such products contain lysine as the active (effective) ingredient, which may be in the salt form with glutamic acid, and additionally contain arginine (including the form of salt(s)).

Further, such products when contain inorganic matters such as calcium are preferable for use as agents against (for opposing) stress-induced diseases.

The inventive products can be used widely for stress-induced diseases. Because the inventive products have great preventive effects, in particular, the inventive products are preferably incorporated during or before stress loading. Consequently, the inventive products can exert the preventive effects. Even if a stress-induced disease occurs, the inventive products can ameliorate or therapeutically treat the disease very readily.

A subject to which the inventive products can be applied includes but is not limited to any animal needing agents against (for opposing) its stress-induced disease. Not only humans but also other animals, particularly cattle and animals for fishery and culture are also included. Before or during stress loading, the inventive products are effectively incorporated, preferably. As to the feeds, animals are the applicable subjects. For various species of animals, particularly cattle and animals for fishery and culture, for example pig, chicken and culture fish, the inventive products are preferably given during or before the loading of stresses during for example transport or shipping. Furthermore, the inventive products are preferable for stresses in case of feeding or culture at higher densities in small feeding lots or culture lots.

For the prevention of stress-induced diseases derived from lysine deficiency, the inventive products can preferably be used. For stress-induced diseases never derived therefrom, the inventive products can be used as well.

The term stress-induced disease is the generic name of diseases caused by psychological stressors and includes all of so-called psychosomatic diseases in addition to the various diseases described above.

The various stress-induced diseases with which the invention is concerned are: anxiety disorders, psychosomatic disorders, dissociated disorders, emotion disorders, abnormalities in gastric motion (abnormal gastric (digestive) motion), irritable colon syndrome (irritable bowel syndrome) and gastric (digestive organ) ulcer.

Lysine is preferably incorporated so that the intake of the lysine may be about (approximately) 0.001 to 13 g/kg/body weight daily, preferably about (approximately) 0.01 to 6.5 g/kg/body weight daily on a lysine free form basis.

Because lysine can be incorporated from those other than the inventive products, the lysine intake from the inventive products is preferably controlled in view of the total lysine intake. In this case, lysine is preferably incorporated from the inventive products so that the total lysine intake may be about (approximately) 0.001 to 13 g/kg/body weight daily, preferably about (approximately) 0.01 to 6.5 g/kg/body weight daily on a free form basis.

The daily lysine intake described above is commonly applicable to pharmaceutical products, foods or drinks and feeds. As described below concerning the upper limit, less lysine intake than the daily lysine intake is recommended, which is applicable only to pharmaceutical products or foods or drinks for humans.

The intake of the lysine may be about (approximately) 0.001 to 1.0 g/kg/body weight daily, preferably about (approximately) 0.01 to 0.5 g/kg/body weight daily on a free form basis. The intake is effective particularly for lysine-deficient humans.

Because lysine can be incorporated from those other than the inventive products as described above, the lysine intake from the inventive products is preferably controlled in view of the total intake of lysine. In this case, the lysine is preferably incorporated from the inventive products, so that the total intake of lysine may be about (approximately) 0.001 to 1.0 g/kg/body weight daily, preferably about (approximately) 0.01 to 0.5 g/kg/body weight daily on a free form basis.

Any content of the lysine in pharmaceutical products, foods or drinks or feeds is satisfactory, with no specific limitation. Depending on the type or form of such product, the content of the lysine can be selected appropriately, which is about (approximately) 90 to 0.1 % (by weight), preferably about (approximately) 10 to 1 % (by weight) on a free form basis.

Any form of the inventive products is satisfactory with no specific limitation. In case of pharmaceutical composition, for example, granules, tablets, infusions, injections and the like are selected. In case of foods or drinks, for example, forms of drinks, nutritious agents and health foods in addition of granules and tablets can be suggested. In case of feeds, forms of lysine applicable to feeds are satisfactory, including for example lysine mixed with feeds of normal forms.

As the other components described above, further, inorganic matters such as vitamin and/or calcium can be added and used, on a needed basis.

### Brief Description of the Drawings

[Fig. 1]

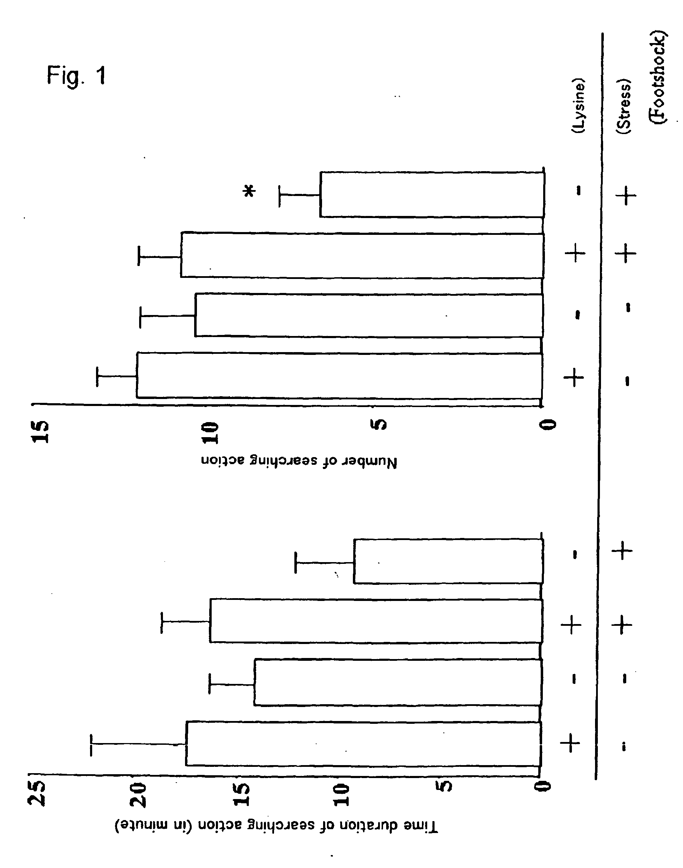

   Fig.1 depicts the time duration how long rats stayed in the box and the number of searching actions, in Example 1. (Lysine) +: lysine-added diet; -: low lysine diet. (Stress) +: under stress loading; -: no stress loading *: p < 0.05 vs. lysine-added diet/ no stress loading and vs. lysine-added diet/ under stress loading.
[Fig. 2]

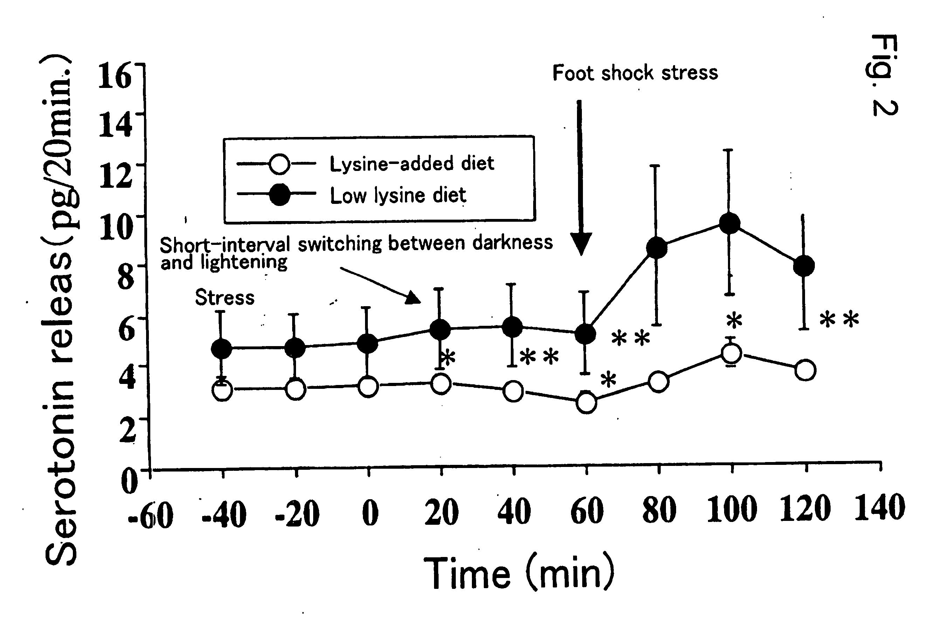

   Fig.2 depicts the change of serotonin concentration in rat brain (amygdala) in Example 1.
   Solid circle: serotonin release from rat on low lysine diet Open circle: serotonin release from rat on lysine-added diet supplemented with lysine deficiency
   *: p < 0.05; **: p < 0.01 vs. lysine-added diet
[Fig. 3]

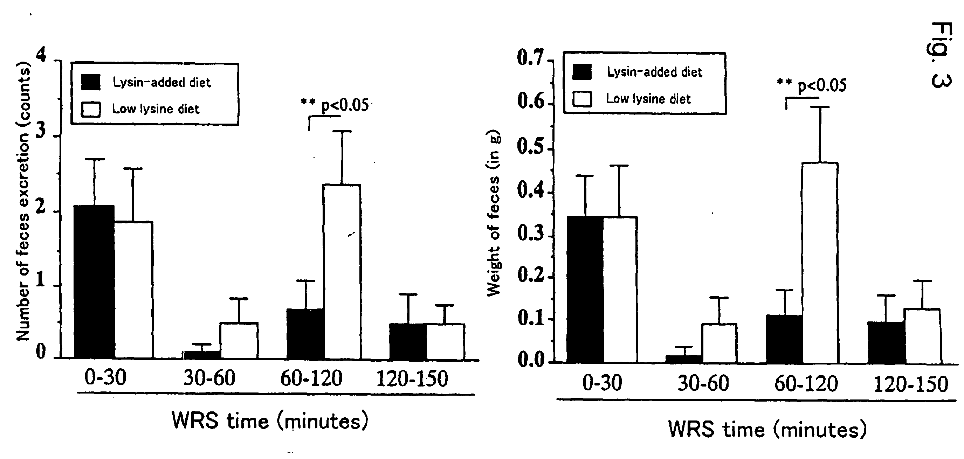

   Fig.3 depicts the number of feces excretion and the weight of feces from rats after wrap stress loading in Example 2.
[Fig. 4]

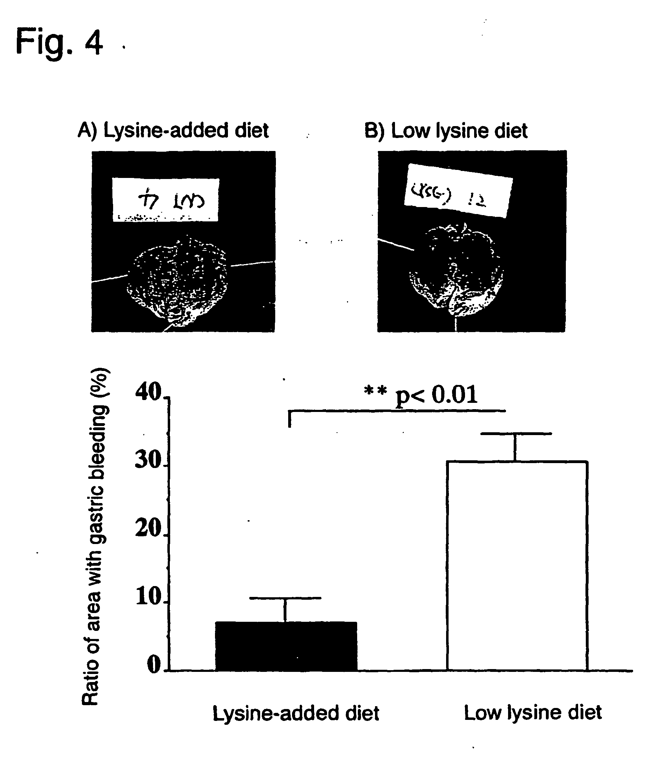

   Fig.4 depicts the area of gastric bleeding in rats and the photopicture thereof in Example 3.
   (Graph) Solid square: lysine-added diet; open square: low lysine diet
   (Photopicture) A) lysine-added diet; B) low lysine diet
[Fig. 5]

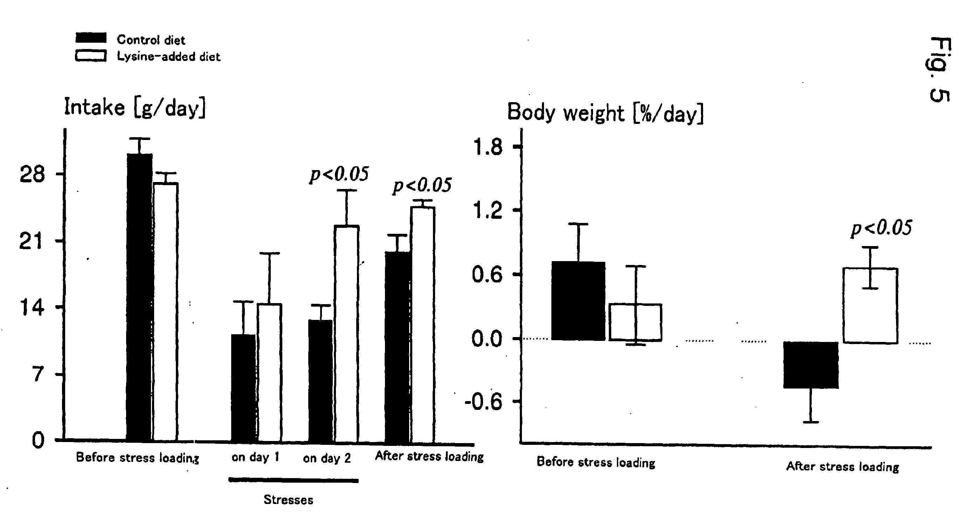

   Fig.5 depicts the changes of total feed intake and body weight over time (days) in rats in Example 4.
   Solid square: control diet (normal diet); open square: lysine-added diet
[Fig. 6]

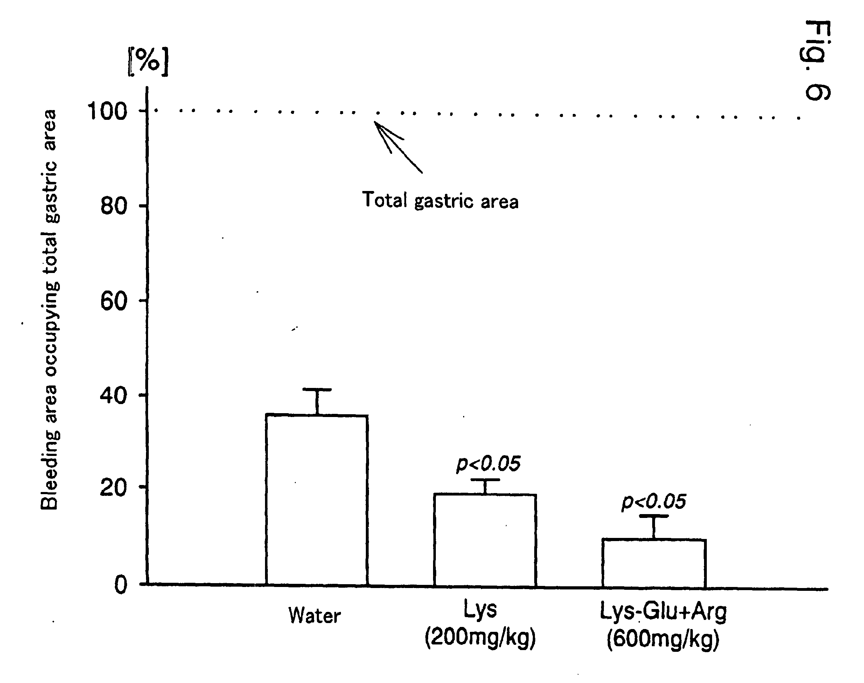

   Fig.6 depicts the results of experiments on stress-induced gastric ulcer in rats in Example 5.
[Fig. 7]

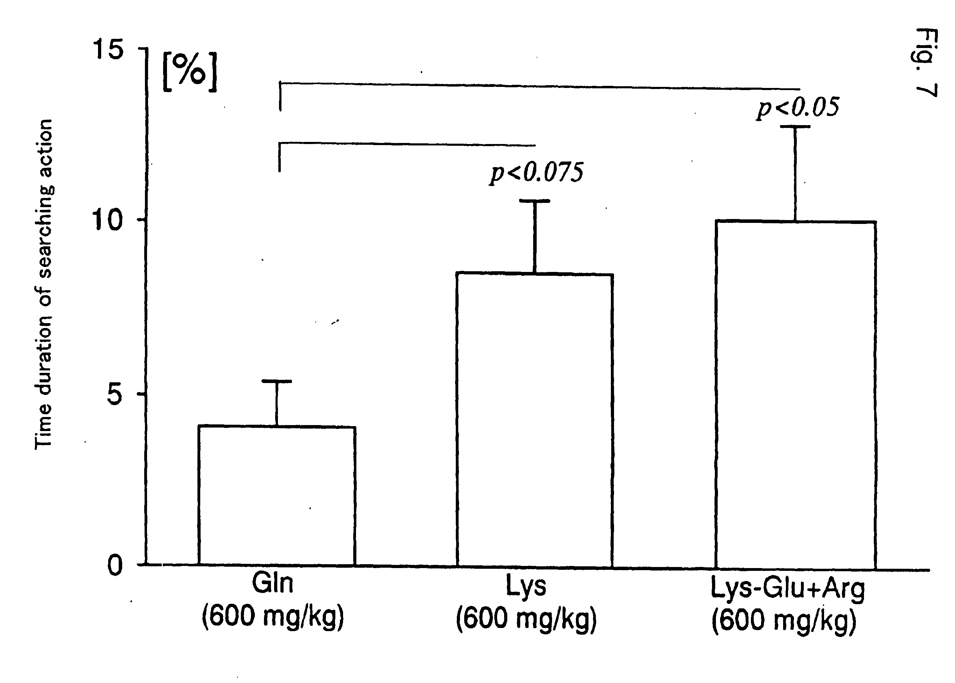

   Fig.7 depicts the results of experiments on the time duration of searching action in rats in Example 6.
[Fig. 8]

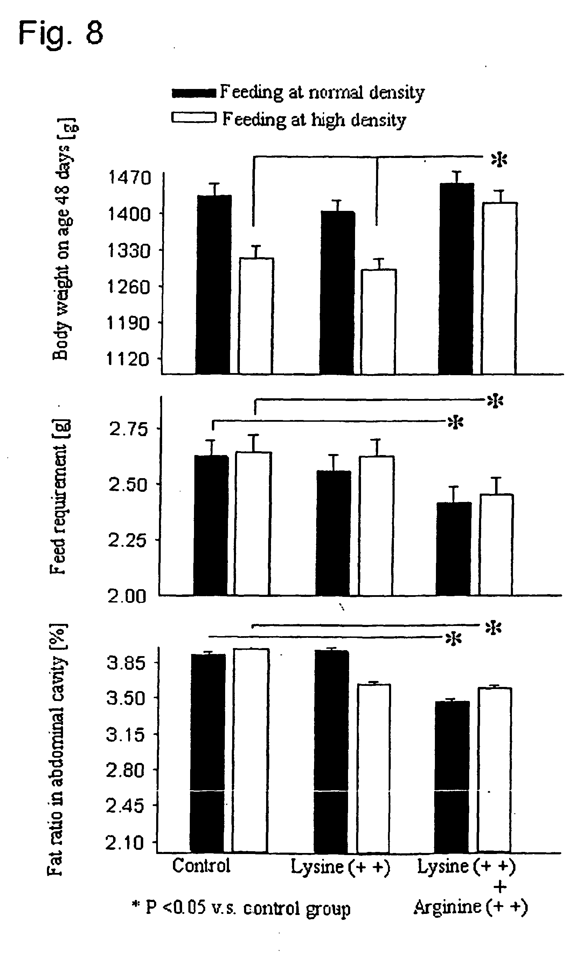

   Fig.8 depicts the results of feeding at high density in broilers in Example 8.
   Solid square: feeding at normal density; open square: feeding at high density; *: p < 0.05 vs. control group.

### Mode for Carrying out the Invention

The mode for carrying out the present invention is now described below.

As described above, in one embodiment, the invention relates to the manufacture of lysine-containing pharmaceutical compositions, foods or drinks or feeds for opposing stress-induced diseases. Thus, the invention is concerned with four types of composition, namely pharmaceutical composition, food, drink and feed, where the content of lysine therein is the active (effective) ingredient. Because the type and form of a final product varies, the difference in terms of these variations should be considered. Unless otherwise stated concerning the difference, however, descriptions in this specification (description) are done in common to the inventions of the four types.

The subject to which the inventive products are given (via eating and drinking or other form of administration includes but is not limited to any of such various animals as described above (humans, cattle, animals for fishery and culture, and other animals with a possibility of the onset of stress-induced diseases), which needs the prevention, amelioration and /or therapeutic treatment and/or the like of stress-induced diseases. Generally, however, the inventive products are applied to mammals, particularly humans (for the feed, the subject includes animals, particularly cattle and animals for fishery and culture (fishes and the like)).

The onset of stress-induced diseases due to lysine deficiency may possibly be caused when dietary life with the main diet corn with a low content of lysine and at a low level of lysine intake from other foods is continued. In case of unbalanced dietary life in aged people and excessively unbalanced nutrition due to extremely low dietary intake in juvenile and young people, they fall into lysine deficiency. Thus, it is anticipated that they readily cause the onset of stress-induced diseases. For people on such a dietary life, lysine of preferably about (approximately) 0.001 to 1.0 g /kg/body weight, more preferably about (approximately) 0.01 to 0.5 g/kg/body weight as daily intake on a free form basis is formulated into dosage forms such as granules and powders and is then given (administered), so that the onset of stress-induced diseases can be prevented effectively.

In an environment with no essential lysine deficiency, further, sufficient intake of lysine from the inventive products preliminarily (before stress loading) or in stressful environment can give the preventive effect. Even at the onset of stress-induced diseases, the stress-induced diseases can readily be cured. Then, the inventive products may be given so that the total intake of lysine in that case may be preferably about (approximately) 0.001 to 1.0 g/kg/body weight, more preferably about (approximately) 0.01 to 0.5 g/kg/body weight daily on a free form basis.

For feeds for animals, the upper limit (for humans) of the amount of lysine for use therein can be elevated, while the lower limit thereof remains as it is. The range of preferable numerical figures for feeds is shown below.

On a free form basis, lysine is given at an amount of preferably about (approximately) 0.001 to 13 g/kg/body weight, more preferably about (approximately) 0.01 to 6.5g/kg/body weight daily on a free form basis. When lysine is given from other sources, lysine is blended in feeds, so that the total intake of lysine may be preferably about (approximately) 0.001 to 13 g/kg/body weight, more preferably about (approximately) 0.01 to 6.5g/kg/body weight daily on a free form basis. The resulting feeds may satisfactorily be given to intended animals.

Compared with the conventional amount of lysine in blend in feeds, the content of lysine in blend in feeds is preferably fairly high so as to securely attain the intended effects sufficiently. It is recommended that lysine for example at an amount 1.1-fold to 3.0-fold the recommended nutritious requirement of lysine (see NRC, the Nippon feeding standards and the like) is to be blended. Even in this case, lysine may be used in the form of salt(s).

The most appropriate lysine intake currently proposed varies, depending on the animal species and the growth stage thereof. For example, the lysine intake calculated on the basis of the recommended value by the National Research Council (NRC), USA is as follows.
Pig: 790 mg/kg/day (for 5-10 kg body weights); 160 mg/kg/day (for 80-100 kg body weights)
Broiler: 1360 mg/kg/day (for age one week after birth); 560 mg/kg/day (for age 8 weeks after birth)
Fish (trout) : 900 mg/kg/day (for 1-2g body weights); 160 mg/kg/day (for 40 g body weights)

The numerical figures of each animal above show the data at its small-size stage and at its large-size stage. At the stage between these stages, the ntermediate numerical figures between these values are suggested.

For formulation of the inventive products into dosage forms for pharmaceutical products, sweeteners and flavor can be added to improve the taste and flavor. The dosage forms are not limited to them. Any dosage form may be satisfactory, including for example liquids such as suspensions and syrups. Even in this case, additionally, sweeteners and flavor can be added to improve the taste and flavor, as described above. If necessary, vitamin and inorganic matters may also be added thereto. The inventive products may be formulated into injections for intravenous administration. For producing these dosage forms, the techniques generally used in the pharmaceutical industry can be used for ready production thereof.

In case of foods or drinks, appropriate amounts of lysine can be added and mixed at the stage of or after the production of various foods or drinks, depending on the type and form of each of the foods or drinks. The inventive products can be prepared into foods or drinks supplemented with a higher amount of lysine. For example, lysine is preliminarily added to cereals at small contents of lysine, such as corn. When the resulting cereals are then incorporated as main diet, the onset of stress-induced diseases can significantly be suppressed. As the intake, lysine at preferably about (approximately) 0. 001 to 1.0 g/kg/body weight, more preferably about (approximately) 0.01 to 0.5 g /kg/body weight daily is appropriate on a free form basis. More preferably, lysine is incorporated from the inventive products, so that the total lysine intake per day may be within the range of the numerical figures described above.

Feeds can be readily prepared according to the already known technique for lysine-supplemented feeds, more preferably by blending lysine set at a higher content level as described above.

Furthermore, it is effective to increase the intake of lysine in normal subjects or patients affected with stress-induced diseases or potential patients with such diseases, by allowing them ingest lysine in the forms of foods or drinks, medical foods or health foods containing lysine as the effective (active) ingredient. As described above, the inventive products are effective, when they are given before or during stress loading for the purpose of preventing thereof. The inventive products are particularly effective as preventive tools of stress-induced diseases due to lysine deficiency.

As described above, in an additional aspect, the invention relates to a method for suppressing stress(anti-stress method), including lysine incorporation (ingestion) in or administration to biological organism(s) (living body(ies)); and in a still additional aspect, the invention relates to a use of lysine in (for) anti-stress agents or production thereof. In these cases, lysine may be in the form of salt(s).

These inventions can be carried out readily, on the basis of the descriptions about the individual pharmaceutical compositions, the foods or drinks, the feeds and the like in accordance with the invention or the descriptions about the agent against (for opposing) stress-induced diseases in accordance with the invention, the after described Examples and the like, with reference to known art, if necessary.

### Preferable Mode for Carrying Out the Invention

The present invention is specifically described in the following examples. However, the invention is not limited to the following examples. Herein, amino acids used in the examples are all in their L forms.

### (Example 1) (Model for assessing anxiety disorders)

A low lysine diet was prepared, using wheat gluten at a small lysine content as the main raw material. This contains lysine at an amount 1/4-fold the ideal lysine requirement and is a lysine-containing diet which can maintain the increase of rat body weight. On the other hand, lysine was added to the low lysine diet to the ideal requirement level of lysine, to prepare a lysine-added diet (so as to prepare the nitrogen source uniform, lysine was added instead of glutamine, at an amount corresponding to the amount of glutamine). Table 1 shows the compositions of the low lysine diet and the lysine-added diet used in this experiment. After 2-week feeding on each diet, Wistar rats (male; age 5 weeks) were used (each group of 6 rats; n= 6). Elevated T-maze test was done with reference to the method of Graeff, et al.

A T maze was arranged at a height of 0. 9 m above the ground surface so that one part might be a box while the remaining two parts might be in an open environment. First, the rats were placed in the box, and the following searching actions thereof were observed through a television monitor. Difference in the time duration and number of their searching actions at such a simple elevated T-maze test was not observed between the rats on the lysine-added diet and the rats on the low lysine diet. When mild stress (foot shock stress) was loaded immediately before the start of the experiment, however, the time duration and number of the searching actions in the rats on the lysine-added diet were not decreased under observation. In the rats on the low lysine diet, nonetheless, the time duration and number thereof were significantly decreased (p < 0.05) (see Fig.1). In other words, their anxiety symptoms were exacerbated. The serotonin concentration in the rat brain (amygdala) after foot shock was assayed over time by the microdialysis method. Then, it was found that the serotonin concentration in the rats on the low lysine diet was significantly increased, compared with the rats on the lysine-added diet (see Fig.2). Amygdala functions as the center of affections such as emotion, while serotonin is generally believed to be one of the transmitters. These indicate that lysine deficiency enhances anxiety state, as taught by behavioral science and neurochemically. Thus, it is indicated that lysine supplement in diet can ameliorate such a state.

**[Table 1]**

| | Low lysine diet (composition ratio in %) | Lysine-added diet (composition ratio in %) |
|---|---|---|
| Corn starch | 20.16 | 19.89 |
| Gluten Mix *1 | 28.07 | 28.07 |
| Pre Mix45 *2 | 45 | 45 |
| Vitamin E | 0.01 | 0.01 |
| Corn Oil | 5 | 5 |
| L-Lysine | 0 | 1.35 |
| L-Glutamine | 1.76 | 0.68 |
| Total | 100 | 100 |

| | | |
|---|---|---|
| *1: see Smriga, et al., J. Nutrition 130, 1641-1643, 2000 *2: (Composition) Starch at 79.6 %, cellulose at 8.9 %, inorganic mixture at 8.9 %, vitamin mixture at 2.2 % and choline C1 at 0.4 %. | | |

These contents are also applied below in Table 2.

### (Example 2) (Model of irritable colon syndrome)

Many people have some experiences such that once on train or on schedule before presentation in an important meeting, they want to rush into toilets. This is the result of activation of gastric motion, particularly bowel motion (motion for feces excretion), which is caused by psychological stresses. On stronger stress, they fall into diarrhea-like symptoms. People with severe such symptoms are called irritable colon syndrome and need therapeutic treatment.

It is known that on mild stress, the feces excretion motion of small animals such as rats is activated. Specifically, both the forefeet of a rat are fixed around its body with tape (wrap stress) to block spontaneous action. Then, the amount of feces is observed. This is called wrap stress resistant model (WRS) and is used for screening pharmaceutical products for the purpose of the therapeutic treatment of the irritable colon syndrome.

Wistar rats (male; age 5 weeks) were fed with the lysine-added diet and the low lysine diet (lysine deficient diet; see Table 1) for 2 weeks (each group of 10 animals). WRS model was prepared by the method of Ito C. (J. Pharmacol. Exp. Ther., 1997, 280, 67-72) and Kishibayashi N. (Jpn. J. Pharmacol . , 1993, 63, 495-502), et al. Specifically, the forefeet of rat were fastened around its body with a cotton tape. The rat was then left in a feeding bracket cage. The excreted feces were collected at an interval of 10 minutes. Fig.3 shows the results of the number of feces excretion and the weight of the excreted feces after collected and integral calculation at a 30-min interval, starting the loading of wrap stress to 150 minutes later. Both the rats on the lysine-added diet and the rats on the low lysine diet had peaks of their actions for feces excretion, within 30 minutes after the start of WRS. Subsequently, the actions were gentler with a peak indicating gentle action for feces excretion over one to 2 hours. Thus, their actions involved two-phase reactions. Between both the groups, no difference was observed in terms of the number of feces excretion and the weight of excreted feces as caused by immediate stress-induced bowel motion as observed in the phase I. However, both the number of feces excretion and the weight of excreted feces due to gradual stress-induced bowel motion as observed in the phase II appearing in the delayed stage were significantly larger in the rats on the low lysine diet (p < 0.05). In other words, lysine deficiency enhances feces excretion due to psychological stress, which is ameliorated by the intake of the lysine-added diet.

### (Example 3) (Model of stress-induced gastric ulcer)

Rats were fed with the low lysine diet and the lysine-added diet supplemented for lysine deficiency (see Table 1) for 3 weeks. After starvation for 18 hours, the rats were placed in a stress gage (Natsume Seisakusho; KN-468) to immerse the rats in water (temperature of 22 to 25 °C) so as to soak their breast for 6 hours. Thereafter, a stomach was resected, to calculate the area with gastric bleeding, to examine the degree of gastric bleeding with the NHI image software. Fig.4 shows the results. Compared with the rats fed with the lysine-added diet, the rats fed with the low lysine diet had significantly increased the degree in areas of gastric bleeding (the ratio of the area of gastric bleeding to the total gastric area) due to the water-immersion restraint. A correlation between gastric bleeding and the incidence of gastric ulcer is observed in this model. In other words, it can be determined that stress-induced gastric ulcer occurring on the low lysine diet can be prevented by adding lysine to diet.

### (Example 4) (Effect of lysine-added diet in model with stress-induced appetite loss)

Wistar rats (male; age 13 weeks; about 400 g or so; N=16) were used at this experiment. 4 days before the start of the experiment and throughout the stress experiment, the rats were fed with normal diet (control diet; lysine at 13.4 g/kg) and the lysine-added diet (27 g/kg). Under conditions of water ad libitum, feeding was done from 9:00 to 11:00. The rats were subjected to foot shock stress (1 mA/3 minutes; once per one hour at 19:00 to 7:00 on the next day) on day 4 of feeding; foot shock stress (1 mA/3 minutes, once per 2 hours at 19:00 to 7:00 on the next day) on day 5; and foot shock stress (1 mA/3 minutes, once at 7:00) on day 6. The change in the total feed intake each day and the change of body weight over days were measured. The results are shown in Fig.5.

Table 2 below shows the compositions of the control diet and the lysine-added diet used herein.

**[Table 2]**

| | Control diet (composition ratio in %) | Lysine-added diet (composition ratio in %) |
|---|---|---|
| Corn starch | 19.89 | 18.54 |
| Gluten Mix | 28.07 | 28.07 |
| Pre Mix45 | 45 | 45 |
| Vitamin E | 0.01 | 0.01 |
| Corn oil | 5 | 5 |
| L-Lysine | 1.35 | 2.70 |
| L-Glutamine | 0.68 | 0.68 |
| Total | 100 | 100 |

The above results indicate that the lysine administration before stress loading allows for the exertion of the preventive effect and suggest also the effect of lysine administration on the amelioration and therapeutic treatment of stress-induced diseases after their onset. In other words, lysine is more preferably administered before stress loading from the prophylactic standpoint. After the onset, lysine administration can make recovery readier. Even in this case, lysine administration before stress loading can enhance the effect more.

### (Example 5) (stress-induced gastric ulcer experiment)

For this experiment, 7-week Wistar rats (male; Nippon Charles-River) were used. For 7 days, normal diet (CRF-1; Oriental Yeast) was fed ad libitum to the rats, for preliminary feeding. For the stress experiment, then, the rats were divided into the following three groups for feeding for another 3 days under starvation conditions: 1) a control group (N=8) fed with pure water; 2) a group (N=8) given with an aqueous lysine solution (40 mg/mL) ; and 3) a group (N=4) given with an aqueous mixture solution of lysine glutamate salt (40 mg/mL) and arginine (40 mg/mL). These aqueous solutions were given to the individual groups in such a manner that dosing of 5 mL of each solution was enforced with an oral probe, once daily at 9:00 in the morning.

During the term, water was fed ad libitum. On day 4, the rats were placed in a stress gage (Natsume Seisakusho; KN-468) as in Example 3, to immerse the rats in water (temperature of 22 to 25 °C) so as to soak their breast for 5 hours to induce stress-induced gastric ulcer. The results are shown in Fig.6. Compared with the control group fed with pure water, the aqueous lysine solution group and the group fed with the aqueous mixture solution of lysine glutamate salt and arginine were observed to have significant reduction of the areas of gastric bleeding. This hopefully suggests that addition of lysine in the form of aqueous solution can prevent stress-induced diseases even under feeding with normal diet.

### (Example 6) (Effect of lysine and lysine-containing amino acid dosage form at elevated T-maze test) [Method]

At the experiment, 7-week Wistar rats (male;, Nippon Charles-River) were used. For 7 days, the normal diet (CRF-1; Oriental Yeast) was fed ad libitum to the rats, for preliminary feeding. For the stress experiment, the rats were divided into the following three groups for feeding for another 3 days under starvation conditions: 1) a group (N=14) fed with an aqueous glutamine solution (120 mg/mL); 2) a group (N=14) given with an aqueous lysine solution (120 mg/mL); and 3) a group (N=14) given with an aqueous mixture solution of lysine glutamate salt (120 mg/mL) and arginine (120 mg/mL).The aqueous solutions were given to the individual groups in such a manner that dosing of 5 mL of each solution was enforced with an oral probe, once daily at 9:00 in the morning. During the term, water was fed ad libitum. On day 4, the rats were placed in a stress gage (Natsume Seisakusho; KN-468) to immerse the rats in water (temperature of 22 to 25 °C) so as to soak their breast for 4 hours for stress loading. Thereafter, elevated T-maze (see Example 1) test was done, for counting the time duration for searching action.

### [Results]

The results are shown in Fig.7. Compared with the glutamine group, the lysine group and the group fed with the aqueous mixture solution of lysine glutamate salt and arginine had significantly prolonged time duration for searching action. Additionally, a group fed with physiological saline and a group fed with glutamine were prepared, to evaluate the effect of glutamine. No significant difference was observed between the two groups. The results described above verified that the dosage form containing lysine alone or the lysine-containing amino acid dosage form prevented anxiety and hypersensitivity after stress loading.

### (Example 7) (Stress from feeding at high density during fishery and culture)

A report tells that the lysine requirement for mojyako (the other name of young yellow tail) according to nutrition science is at 1.78 % in dried feed (see T. Ruchimat et al., Aquaculture 158 (1997), 331-339).

Generally, lysine of an amount exceeding the requirement even when given cannot improve the result of feeding. In a circumstance under stress loading, however, lysine when administered at a level excessive from the standpoint of nutrition science can prevent the reduction of feed intake due to stress, which consequently improves the feeding results. The following experiment is one of the examples.

Test feeds were prepared by adding lysine hydrochloride salt at the ratio shown in Table 3 below to a commercially available mashed feed for mojyako. The lysine content in the dry feed is thereby at 4.8 %, which is far above the dietetic requirement (1.78 %). For comparison, further, a feed was prepared by adding arginine hydrochloride salt at the same level instead of lysine hydrochloride salt, for use at the test.

As test fish, each group of 40 mojyako fishes of mean fish body weight of about 35 g were placed in one 800-L water tank, to which the feeds were fed two times daily at 8: 00 in the morning and 16:00 in the afternoon to satiation, for a total of 4 weeks. During the term, their body weights were measured weekly. Herein, feeding was not done at 16:00 in the afternoon on the day before the day for body weight measurement.

**[Table 3]**

| Composition of feed blends | | | |
|---|---|---|---|
| Test feeds | Control | Arginine added | Lysine added |
| Mashed feed | 860 | 820 | 820 |
| Fish oil | 140 | 140 | 140 |
| Water | 400 | 400 | 400 |
| Arginine HCl | 0 | 40 | 0 |
| Lysine HCl | 0 | 0 | 40 |
| Moisture (%) | 32.4 | 30.8 | 31.7 |
| Protein (%) | 25.7 | 28.5 | 28.2 |
| Lysine (% in dry weight) | 1.8 | 1.8 | 4.8 |
| Lipid (%) | 15.4 | 14.8 | 14.7 |
| Ashes (%) | 7.91 | 7.89 | 7.78 |

The feeding results are shown in Table 4.

**[Table 4]**

| Growth results | | | | |
|---|---|---|---|---|
| Test lots | | Control | Arginine | Lysine |
| Test duration (day) | | 28 | 28 | 28 |
| Feeding duration (day) | | 24 | 24 | 24 |
| Survival rate (%) | | 100 | 100 | 100 |
| Mean body weight | at start | 35.1 | 35.5 | 34.8 |
| | on 1 week | 45.5 | 45.7 | 46.4 |
| | on 2 week | 62.7 | 61.5 | 63.7 |
| | on 3 week | 79.9 | 77.6 | 84.2 |
| | on 4 week (final) | 95.7 | 98.5 | 108.9 |
| Daily increment (%) | | 3.31 | 3.36 | 3.68 |
| Total feed intake (g) | | 3034 | 3072 | 3452 |
| Weekly feed intake (g) | for 0-1 week | 18.6 | 18.7 | 18.4 |
| | for 1-2 week | 37.0 | 36.7 | 38.3 |
| | for 2-3 week | 54.3 | 54.5 | 59.9 |
| | for 3-4 week | 74.0 | 76.8 | 86.3 |
| Daily feeding ratio (%) | | 4.09 | 4.09 | 4.29 |
| Feed efficiency (%) | | 82.9 | 82.0 | 85.8 |

It is assumed that higher stress is loaded to such feeding in water tanks as in this feeding test, compared with normal feeding in sea water. Further, it is also assumed that as the fish body weight increases during the feeding test, stress due to such highly densified feeding in the same volume of a water tank will be intensified. In the lysine lot, the feed intake was larger than those of the remaining experimental lots over the 3 week to 4 week, involving higher body weight increment (at the termination of the test, the increment was 17 %, compared with the control).

Because such effect could not be obtained in the arginine lot, the effect is believed to be inherent to lysine under stress conditions.

### (Example 8) (Stress on broiler from feeding at high density) [Experimental method]

Broiler (species: Arbor Acres) was fed under very hot conditions. The compositions of the feeds are as shown in Tables 5 and 6.

**[Table 5]**

| | Normal diet [kcal/kg] | Lysine-added feed [kcal/kg] | (Lysine+arginine)-added feed [kcal/kg] |
|---|---|---|---|
| Crude protein | 18 | 18 | 18 |
| Crude fat | 6.2 | 6.2 | 6.2 |
| Linoleic acid | 1.91 | 1.91 | 1.91 |
| Crude fiber | 2.38 | 2.38 | 2.38 |
| L-Lysine | 0.85 | 1.7 | 1.7 |
| L-Arginine | 1.18 | 1.18 | 2.36 |
| L-Methionine | 0.32 | 0.32 | 0.32 |
| L-Cysteine | 0.39 | 0.39 | 0.39 |
| L-Threonine | 0.71 | 0.71 | 0.71 |
| Tryptophan | 0.16 | 0.16 | 0.16 |
| L-Serine | 1.62 | 1.62 | 1.62 |
| L-Isoleucine | 0.75 | 0.75 | 0.75 |
| L-Leucine | 1.73 | 1.73 | 1.73 |
| L-Valine | 0.92 | 0.92 | 0.92 |
| L-Glycine | 0.58 | 0.58 | 0.58 |
| Calcium | 0.9 | 0.9 | 0.9 |
| Phosphorus | 0.59 | 0.59 | 0.59 |
| Non-phytin phosphorus | 0.4 | 0.4 | 0.4 |
| Sodium chloride | 0.4 | 0.4 | 0.4 |

**[Table 6]**

| | Control [%] | Lysine added[%] | (Lysine+Arginine) added[%] |
|---|---|---|---|
| Corn | 67.40 | 67.40 | 67.40 |
| Corn starch | 3.00 | 2.42 | 1.82 |
| Soy bean bran | 14.20 | 14.20 | 14.20 |
| Corn gluten meal | 2.50 | 2.50 | 2.50 |
| Fish powder | 1.00 | 1.00 | 1.00 |
| Feather meal | 4.00 | 4.00 | |
| Palm oil | 3.20 | 3.20 | 3.20 |
| Hydrochloric acid L-lysine | 0.59 | 1.17 | 1.17 |
| DL-Methionine | 0.07 | 0.07 | 0.07 |
| L-Arginine | 0.60 | 0.60 | 1.20 |
| L-Tryptophan | 0.01 | 0.01 | 0.01 |
| Dibasic calcium phosphate (CaHPO₄) | 1.15 | 1.15 | 1.15 |
| Calcium carbonate | 1.75 | 1.75 | 1.75 |
| Edible salt | 0.28 | 0.28 | 0.28 |
| Premix | 0.25 | 0.25 | 0.25 |
| Total | 100.0 | 100.0 | 100.0 |

The feeding density was 8 chickens /1 m² (normal density) and 12 chickens / 1 m² (high density) in two lots; the feeds were the following three groups: Feed 1. normal feed; Feed 2. normal feed + lysine (the lysine content 2-fold that in Feed 1.); or Feed 3. normal feed + lysine + arginine (the lysine content and the arginine content were individually 2-fold those in Feed 1.). A total of 6 experimental examples were set as combinations of the feeding densities and the feeds. The test was started on age 21 days (the mean body weight then was 722 g). The feeding results (body weight increment, feed requirement) on age 48 days were compared thereto. On the age 48 days, the chickens were sacrificed to death, for the evaluation of their meat quality (fat ratio in abdominal cavity). The feed requirement was expressed by the feed amount required for the increment of 1 g/body weight, as calculated by dividing the total feed intake by the body weight increment. The feeding results are shown in Fig.8.

### [Results]

In the normal feed group, the reduction of the feed intake and body weight increment in the high density feeding lot was observed, which is presumably ascribed to the stress from the feeding at such high density. In the lysine-added feed group, no improvement of body weight and feed requirement was observed in the normal density feeding lot and the high density feeding lot. However, the fat ratio in abdominal cavity was likely to decrease in the group. In the (lysine + arginine) -added feed group, no body weight increase was observed under feeding at normal density. The results may be due to the reduction of the stress from the feeding at high density, by the feeding of the (lysine + arginine) feed. By comparison in terms of meat quality, on the other hand, the fat ratio in abdominal cavity and the feed requirement were significantly reduced in the (lysine + arginine) -added feed group, so that the improvement of meat quality as well as the improvement of feeding efficiency was observed. The results of these experiments indicate that the amino acid dosage form (lysine + arginine) containing lysine apparently can improve the feeding efficiencies (body weight increase, reduction of feed requirement and reduction of fat ratio in abdominal cavity) of broilers under very hot conditions.

For broilers, the antagonistic action between lysine and arginine is known. A report tells that the ratio of lysine and arginine in feeds should be controlled at an appropriate level. At the experiments, thus, arginine was added in the lysine lot, so that the ratio of lysine to arginine might be constant. In other words, it is known that the ratio of arginine/lysine in the amino acid composition of feeds is very important at the growth stage of broilers for the growth thereof and that a smaller ratio thereof more readily induces growth inhibition. It is reported that the action is prominently great in broiler, compared with mammalian cases, such as rat, dog and pig (see J. Am. Coll. Nutrition 16, 1997, 7-21; J. Nutrition 115, 1985, 743-752; FASEB Special Publications Office, pp 22, 59, 1992). At the present experiment, at least the ratio of arginine/lysine in the feed is lowered via the loading of lysine alone, but no growth inhibition is observed. This may be ascribed to the results of the masking of the body weight increase due to the anti-stress action of lysine with the weight decrease through the reduction of the ratio arginine/lysine, so that it can be said that the effect can substantially be observed.

As described above, it was confirmed that the problem of poor feeding results of broiler due to the stress from the high density feeding could be solved by the addition of lysine/arginine.

### [Technical Background]

So as to provide broiler as a food material of high quality in an inexpensive manner as much as possible, the number of broilers under feeding per feeding area should be elevated, while the feeding efficiency should also be elevated simultaneously. However, feeding of territory-conscious broilers at a certain density or more gives strong stress to the broilers, inducing the decrease of feed intake (so-called stress-induced appetite loss). Additionally, the stress from feeding at high density triggers fighting instinct, so that the chickens injure each other (a phenomenon called so-called cannibalism). Consequently, the body weight increase is delayed, involving the reduction of feed efficiency and therefore the deterioration of meat quality, so that the value as a food material is reduced. The problem of the stress from feeding at high density is more serious under very hot conditions, in particular.

From the standpoint of animal welfare, in recent years, it is demanded that feeding animals for food materials should be fed and kept at a state with stress reduced as much as possible, until the animals are sacrificed to death for meat for food. As the effects on humans of pharmaceutical drugs (antibiotics such as penicillin and a certain type of hormone agents) remaining in meat for food have been elucidated gradually, it is more difficult to formulate synthetic pharmaceutical products with stress-reducing actions for feeding animals, which are to be provided in future as food materials. The inventive technique is industrially very useful in that the use of amino acids (lysine + arginine) can elevate the feeding density of broiler without any occurrence of the problem of the stress from feeding at high density, to provide safe food materials at high productivity to the world.

The results of the above Examples indicate that lysine is effective as an agent against (for opposing) stress-induced diseases, particularly as an agent for preventing stress-induced diseases. It is thus understood that lysine can be used widely in pharmaceutical compositions, foods or drinks or feeds or the like so as to obtain the effect. Additionally, it is understood that the combined use of other specific amino acids, for example glutamic acid and arginine can further enhance the effect.

### Advantages of the Invention

In accordance with the present invention, pharmaceutical products (pharmaceutical compositions), foods or drinks or feed effective for the prevention, amelioration and therapeutic treatment and the like of stress - induced diseases, particularly effective for the prevention thereof can be provided, using lysine as the active (effective) ingredient. Additionally, the invention provides a lysine-containing agent against stress-induced diseases, which can be used in them. In accordance with the invention, further, a method for suppressing stress (anti-stress method) and a use of lysine for these agents or pharmaceutical drugs or the like, or production thereof are also provided.

Thus, the invention is applicable widely in the fields of pharmaceutical products, foods, feeds, clinical practices and the like, and is therefore very useful industrially.

## Claims

1. Use of free lysine, or a salt thereof, as active ingredient in the manufacture of a therapeutic composition against a stress-induced disease selected from anxiety disorders, psychosomatic disorders, dissociated disorders, emotional disorders and diseases attributable to psychological stress selected from abnormal gastric motion, irritable colon syndrome (irritable bowel syndrome) and gastric ulcer, said therapeutic composition being selected from a pharmaceutical composition, a food, a drink, and a feed.

2. Use according to claim 1, wherein the pharmaceutical composition, food, drink or feed is for preventing a stress-induced disease.

3. Use according to claim 1 or 2, wherein the pharmaceutical composition, the food or drink or the feed additionally contains other amino acid(s) (which may be in the form of salt(s)).

4. Use according to any one of claims 1 to 3, wherein lysine is in the form of the salt with glutamic acid or aspartic acid.

5. Use according to claim 3, where the other amino acid(s) include at least one of glutamic acid, arginine and aspartic acid.

6. The use according to claim 3 or 4, where lysine is in the form of the salt with glutamic acid and the pharmaceutical composition, food, drink or feed additionally contains arginine.

7. A use according to any one of the preceding claims, wherein the pharmaceutical composition, the food or drink or the feed is administered during or before stress loading.

8. A use according to any one of the preceding claims wherein the pharmaceutical composition, the food or drink or the feed is for preventing stress-induced diseases due to lysine deficiency.

9. A use according to any one of the preceding claims wherein the pharmaceutical composition, the food or drink or the feed is arranged for a lysine-intake of 0.001 to 13 g/kg/body weight daily on a free lysine form basis.

10. A use according to any one of the preceding claims wherein the pharmaceutical composition, the food or drink or the feed contains the lysine at 90 to 0.1 % by weight on a free form basis.

11. A use according to any one of the preceding claims wherein said composition is a feed which contains lysine at a content 1.1- to 3.0-fold the recommended nutritious requirement of lysine, where the lysine may be in the form of salt(s).

## Patentansprüche

1. Verwendung von freiem Lysin oder eines Salzes davon als aktiver Bestandteil bei der Herstellung einer therapeutischen Zusammensetzung gegen eine stressinduzierte Krankheit, die unter Angststörungen, psychosomatischen Störungen, Doppelbewusstseinszuständen, emotionalen Störungen und Störungen ausgewählt sind, die auf psychologischen Stress zurückzuführen sind und unter abnormaler Darmbewegung, Reizdarmsyndrom und Magengeschwüren ausgewählt sind, wobei die therapeutische Zusammensetzung unter einer Arzneimittelzusammensetzung, einem Nahrungsmittel, einem Getränk und einem Futtermittel ausgewählt ist.

2. Verwendung nach Anspruch 1, wobei die Arzneimittelzusammensetzung, das Nahrungsmittel, das Getränk oder das Futtermittel der Verhinderung einer stressinduzierten Krankheit dient.

3. Verwendung nach Anspruch 1 oder 2, wobei die Arzneimittelzusammensetzung, das Nahrungsmittel oder das Getränk oder das Futtermittel zusätzlich eine oder mehrere andere Aminosäuren enthält (die in der Form eines oder mehrerer Salze vorliegen können).

4. Verwendung nach einem der Ansprüche 1 bis 3, wobei Lysin in der Form des Salzes mit Glutaminsäure oder Asparaginsäure vorliegt.

5. Verwendung nach Anspruch 3, wobei die eine oder mehreren Aminosäuren mindestens eine unter Glutaminsäure, Arginin- und Asparaginsäure ausgewählte Aminosäure umfassen.

6. Verwendung nach Anspruch 3 oder 4, wobei Lysin in der Form des Salzes mit Glutaminsäure vorliegt und die Arzneimittelzusammensetzung, das Nahrungsmittel, das Getränk oder das Futtermittel zusätzlich Arginin enthält.

7. Verwendung nach einem der vorstehenden Ansprüche, wobei die Arzneimittelzusammensetzung, das Nahrungsmittel oder Getränk oder das Futtermittel während oder vor der Stressbelastung verabreicht wird.

8. Verwendung nach einem der vorstehenden Ansprüche, wobei die Arzneimittelzusammensetzung, das Nahrungsmittel oder Getränk oder das Futtermittel der Verhinderung von stressinduzierten Krankheiten auf Grund von Lysindefizienz dient.

9. Verwendung nach einem der vorstehenden Ansprüche, wobei die Arzneimittelzusammensetzung, das Nahrungsmittel oder Getränk oder das Futtermittel für eine tägliche Lysinaufnahme von 0,001 bis 13 g/kg/Körpergewicht, auf Basis der freien Lysinform, bereitgestellt wird.

10. Verwendung nach einem der vorstehenden Ansprüche, wobei die Arzneimittelzusammensetzung, das Nahrungsmittel oder Getränk oder das Futtermittel das Lysin in einer Menge von 90 bis 0,1 Gew.-%, auf der Basis der freien Form, enthält.

11. Verwendung nach einem der vorstehenden Ansprüche, wobei die Zusammensetzung ein Futter ist, das Lysin in einer Menge des 1,1- bis 3,0-fachen des empfohlenen Nährstoffbedarfs an Lysin enthält, wobei das Lysin in der Form eines oder mehrerer Salze vorliegen kann.

## Revendications

1. Utilisation de la lysine libre, ou d'un sel de celle-ci, comme ingrédient actif dans la fabrication d'une composition thérapeutique contre une maladie induite par le stress choisie parmi les troubles de type angoisse, les troubles psychosomatiques, les troubles dissociés, les troubles émotionnels et les maladies attribuables à un stress psychologique choisies parmi un mouvement gastrique anormal, le syndrome du côlon irritable (syndrome de l'intestin irritable), et l'ulcère gastrique, ladite composition thérapeutique étant choisie parmi une composition pharmaceutique, un aliment, une boisson et un aliment pour animaux.

2. Utilisation selon la revendication 1 où la composition pharmaceutique, l'aliment, la boisson ou l'aliment pour animaux est destinée à prévenir une maladie induite par le stress.

3. Utilisation selon la revendication 1 ou 2 où la composition pharmaceutique, l'aliment ou la boisson ou l'aliment pour animaux contient en outre un ou plusieurs autres aminoacides (qui peuvent être sous forme de sel(s)).

4. Utilisation selon l'une quelconque des revendications 1 à 3 où la lysine est sous forme du sel avec l'acide glutamique ou l'acide aspartique.

5. Utilisation selon la revendication 3 où le ou les autres aminoacides incluent au moins un parmi l'acide glutamique, l'arginine et l'acide aspartique.

6. Utilisation selon la revendication 3 ou 4 où la lysine est sous forme du sel avec l'acide glutamique et la composition pharmaceutique, l'aliment, la boisson ou l'aliment pour animaux contient en outre de l'arginine.

7. Utilisation selon l'une quelconque des revendications précédentes où la composition pharmaceutique, l'aliment ou la boisson ou l'aliment pour animaux est administrée pendant ou avant une charge de stress.

8. Utilisation selon l'une quelconque des revendications précédentes où la composition pharmaceutique, l'aliment ou la boisson ou l'aliment pour animaux est destinée à prévenir les maladies induites par le stress dues à une déficience en lysine.

9. Utilisation selon l'une quelconque des revendications précédentes où la composition pharmaceutique, l'aliment ou la boisson ou l'aliment pour animaux est agencée pour une absorption de lysine de 0,001 à 13 g/kg/poids corporel quotidiennement sur une base de forme de lysine libre.

10. Utilisation selon l'une quelconque des revendications précédentes, où la composition pharmaceutique, l'aliment ou la boisson ou l'aliment pour animaux contient la lysine à raison de 90 à 0,1 % en masse sur une base de forme libre.

11. Utilisation selon l'une quelconque des revendications précédentes où ladite composition est un aliment pour animaux qui contient de la lysine à une teneur de 1,1 à 3,0 fois les besoins nutritionnels de lysine recommandés, où la lysine peut être sous forme de sel(s).
